# FASCICULE DE BREVET EUROPEEN

(11) **EP 1 283 911 B1**
(45) Date de publication et mention de la délivrance du brevet: **04.07.2007**
(21) Numéro de dépôt: 01938324.9
(22) Date de dépôt: 23.05.2001
(51) Int. Cl.: C12Q 1/68, G01N 33/68

(54) **DETECTION ET CARACTERISATION DE L'ACTIVITE DE PROTEINES IMPLIQUEES DANS LA REPARATION DE LESIONS DE L'ADN**
DETEKTION UND CHARAKTERISIERUNG DER AKTIVITÄT VON PROTEINEN, WELCHE AN DER REPARATUR VON DNA-LÄSIONEN BETEILIGT SIND
METHOD FOR DETECTING AND CHARACTERISING ACTIVITY OF PROTEINS INVOLVED IN LESION AND DNA REPAIR

(30) Priorité: 24.05.2000 FR 0006624
(43) Date de publication de la demande: 19.02.2003
(73) Titulaire: COMMISSARIAT A L'ENERGIE ATOMIQUE, 75015 Paris (FR)
(72) Inventeur: SAUVAIGO, Sylvie, F-38320 Herbeys (FR)
(74) Mandataire: Audier, Philippe André
(86) Numéro de dépôt international: PCT/FR2001/001605
(87) Numéro de publication internationale: WO 2001/090408

(56) Documents cités:
- FR-A- 2 731 711
- FR-A- 2 763 600
- SALLES B ET AL: "A CHEMILUMINESCENT MICROPLATE ASSAY TO DETECT DNA DAMAGE INDUCED BYGENOTOXIC TREATMENTS" ANALYTICAL BIOCHEMISTRY,US,ACADEMIC PRESS, SAN DIEGO, CA, vol. 232, 1995, pages 37-42, XP002054956 ISSN: 0003-2697
- SALLES B ET AL: "IN VITRO EUKARYOTIC DNA EXCISION REPAIR ASSAUS: AN OVERVIEW" BIOCHIMIE,FR,PARIS, vol. 77, no. 10, 1995, pages 796-802, XP002054957 ISSN: 0300-9084 cité dans la demande
- D'HAM C ET AL.: "Excision of 5,6-dihydoxy-5,6-dihydrothymine, 5,6-dihydrothymine, and 5-hydroxycytosin from defined sequence oligonucleotides by Escherichia coli endonuclease III and fpg proteins: Kinetic and mechanistic aspects" BOCHEMISTRY, vol. 38, 1999, pages 3335-3344, XP002164194 cité dans la demande
- CALSOU P ET AL: "MEASUREMENTS OF DAMAGE-SPECIFIC DNA INCISION BY NUCLEOTIDE EXCISION REPAIR IN VITRO" BIOCHEMICAL AND BIOPHYSICAL RESEARCH COMMUNICATIONS,US,ACADEMIC PRESS INC. ORLANDO, FL, vol. 202, no. 2, 29 juillet 1994 (1994-07-29), pages 788-795, XP002008620 ISSN: 0006-291X

## Description

### Domaine technique

La présente invention a pour objet un procédé de détection et de caractérisation de l'activité de protéines impliquées dans la réparation de lésions de l'ADN.

Elle s'applique en particulier à l'étude des enzymes et des facteurs protéiques impliqués dans la réparation de l'ADN, ainsi qu'à l'étude de la génotoxicité de substances chimiques ou d'agents physiques dans des systèmes vivants.

### État de la technique antérieure

Des lésions ou dommages peuvent se former dans l'ADN cellulaire par exposition à divers agents chimiques ou physiques, en particulier après irradiation (rayonnements ionisants et solaires), après induction par un stress oxydant, ou après exposition à certains agents génotoxiques ou cytotoxiques. Des nucléosides endommagés sont également susceptibles de s'accumuler naturellement dans le génome, au cours du processus de vieillissement cellulaire.

Les lésions ou dommages des acides nucléiques sont de plusieurs types et sont souvent caractéristiques de l'agent qui les a induits. Ces dommages incluent des cassures simple et double-brin, des sites abasiques et des modifications de bases nucléiques.

Suivant la nature de la lésion, différents mécanismes de réparation entrent en jeu et peuvent même être en compétition. On peut distinguer trois mécanismes principaux de réparation: la réparation des mésappariements, la réparation par excision de nucléotides (REN) et la réparation par excision de bases (REB). D'autre part, la recombinaison homologue qui est un mécanisme cellulaire intervenant lors de la méiose, permet la réparation des cassures double brins d'ADN.

Ces mécanismes font intervenir différentes protéines de réparation. Ainsi, le mécanisme de réparation par excision de nucléotides (REN) implique plusieurs étapes qui sont :
1) la reconnaissance du dommage par un complexe protéique,
2) l'incision du brin contenant la lésion de chaque coté de celle-ci,
3) l'excision d'un fragment oligonucléotidique contenant la lésion, et
4) la synthèse du nouveau brin d'ADN intègre avec une ligation finale.

De nombreuses protéines et enzymes sont impliquées dans ce mécanisme qui répare principalement les lésions photo-induites et les dommages volumineux provoqués par des traitements chimiques.

Le mécanisme de réparation par excision de bases (REB) fait appel à une famille d'enzymes appelées glycosylases. Celles-ci sont parfois spécifiques du substrat à exciser, mais en général reconnaissent des catégories de modifications. Certaines glycosylases possèdent également des activités endonucléases. Ces enzymes réparent les bases oxydées, fragmentées ou alkylées. Ce système prend en charge, en outre, les sites abasiques, certains mésappariements, certaines cassures simple brin.

Chez les humains, six glycosylases ont été identifiées (Wilson III, D.M. and Thompson, L.H., 1997, Proc. Natl. Acad. Sci., 94, pages 12754-12757 [9]),qui servent principalement à la réparation de bases désaminées, oxydées, alkylées, ou bien à la correction de certains mésappariements. L'action des glycosylases résulte en la formation d'un site abasique reconnu par une endonucléase spécifique qui incise la liaison phosphodiester en 5' de la base éliminée. Une polymérase remplace le nucléotide éliminé et une ligase referme la chaîne nucléotidique. Il est à noter que certaines glycosylases possèdent une activité lyase associée qui incise la liaison phosphodiester en 3' de la lésion et conduit à la formation d'une extrémité 5'-phosphate. Il existe une alternative à cette voie de REB au cours de laquelle un petit fragment d'ADN contenant le dommage est éliminé, puis remplacé. Cela pourrait concerner certaines cassures simple brin de l'ADN. Des enzymes spécifiques codées par des gènes identifiés sont ainsi associées à l'excision des lésions définies.

Les procédés destinés à évaluer les activités de réparation cellulaire sont compliqués et lourds à mettre en oeuvre. Ils nécessitent souvent l'utilisation de plasmides modifiés chimiquement. La capacité de réparation d'extraits cellulaires est mesurée *in vitro* par la réparation de ces modifications corrélée à l'incorporation de nucléotides marqués, lors de la resynthèse des brins excisés. La méthode a été initialement développée par Wood et al., Cell, 53, 1988, pages 97-106 [1]. Les plasmides superenroulés sont modifiés par des rayonnements UV de type C, l'acétyl-aminofluorène ou bien le cis-dichlorodiamnineplatine. Les plasmides sont ensuite incubés dans un milieu contenant des extraits cellulaires, les quatre désoxynucléosidetriphosphates, dont un est marqué en position α : par un ³²P, de l'ATP et un système de régénération d'ATP. Pendant l'incubation, des lésions peuvent être éliminées par les systèmes d'excision de nucléotides ou d'excision de bases. Le taux de resynthèse d'ADN est déterminé après migration de l'ADN sur gel d'agarose et comptage de la radioactivité de la bande recherchée. L'importance et la spécificité de la réparation sont liées à la qualité des extraits et du plasmide, aux lésions créées et aux paramètres de réaction, comme il est décrit par Sallès et al, Biochimie, 77, 1995, pages 796-802 [2]. En particulier, une préparation plasmidique initiale est nécessaire pour éliminer les plasmides contenant des cassures avant traitement ou des fragments de chromosome bactérien qui fourniraient .des sites d'initiation de polymérisation.

Un autre inconvénient de cette technique a trait aux lésions créées. Les traitements utilisés n'induisent pas un défaut unique. Par exemple, les dommages majoritaires engendrés par les rayonnements UVC sont les dimères de pyrimidine de type cyclobutane. Mais d'autres lésions sont formées comme les photoproduits, des cassures de chaînes, des hydrates de cytosine etc. Ainsi, un suivi particulier de la réparation de chacun des dommages ne peut pas être effectué facilement à l'aide de ces substrats.

Le document FR-A-2 731 711 [3] décrit un procédé de détection de lésions de l'ADN consistant à fixer l'ADN sur une phase solide, puis à créer des lésions sur cet ADN au moyen d'un produit lésant et à utiliser des extraits cellulaires contenant des facteurs de réparation et un marqueur. Le but de ce système est de permettre la mesure des effets génotoxiques de certaines substances chimiques. L'ADN sous forme de plasmides est tout d'abord fixé sur une phase solide (puits de microplaque), puis les plasmides sont modifiés chimiquement. Les réactions de réparation sont effectuées en présence de nucléosides triphosphates modifiés permettant une détection non radioactive des brins néo-synthétisés. Ce système ne permet pas d'incorporer de façon ciblée, à l'endroit désiré des modifications spécifiques. Il s'agit d'un système qui permet la détection d'un effet global d'un agent modificateur de l'ADN sans identifier les lésions reconnues par les systèmes de réparation. Par ailleurs, ce procédé n'a pas pour but de détecter et quantifier l'activité des protéines impliquées dans la réparation de l'ADN, mais d'identifier la présence de lésions sur l'ADN traité.

Page et al, Biochemistry, 29, 1990, pages 1016-1024 [4] et Huang et al, Proc. Natl. Acad. Sci., 91, 1994, pages 12213-12217, [5], utilisent un test différent afin de mesurer les capacités d'excision de dommages par des extraits cellulaires de diverses origines. Des lésions spécifiques sont créées ou incorporées dans de courts oligonucléotides de synthèse. Ceux-ci sont ensuite liés entre eux par des ligases pour donner des fragments doubles-brins plus longs (150 à 180 paires de bases). L'incorporation de ³²P en différentes positions des fragments permet de localiser les sites de coupure créés par les enzymes de réparations. L'analyse s'effectue par autoradiographie après électrophorèse sur gel d'acrylamide. Ce test est applicable aux réparations de type REB et REN.

Tous les procédés décrits ci-dessus présentent des inconvénients. Dans la plupart des cas, des marquages radioactifs et des séparations longues par électrophorèse sont nécessaires afin de déterminer l'excision des dommages. L'utilisation de plasmides est lourde à mettre en oeuvre car des précautions sont nécessaires afin de s'assurer de leur pureté et minimiser le bruit de fond. D'autre part, comme on l'a mentionné, l'induction d'un seul type de dommage par un stress est impossible et plusieurs lésions sont ainsi présentes simultanément dans l'ADN.

On connaît aussi des procédés permettant d'étudier les spécificités et les mécanismes d'excision de dommages par des enzymes de réparation sur des oligonucléotides de synthèse de 15 à 50 bases de long contenant des lésions bien définies, comme il est décrit par Romieu et al, J. Org. Chem., 63, 1998, pages 5245-5249 [6] et par D'Ham et al, Biochemistry, 38, 1999, pages 3335-3344 [7].

Dans la plupart des cas, l'oligonucléotide contenant la ou les modifications chimiques à étudier est marqué radioactivement à une de ses extrémités. L'action de l'enzyme (cinétique d'excision, détermination des constantes d'affinité, coupure ou non du fragment oligonucléotidique, action sur un simple ou un double brin) est analysée après électrophorèse sur gel d'acrylamide.

Une autre technique évite l'emploi de la radioactivité mais nécessite un investissement important : c'est l'analyse de la digestion par spectrométrie de masse (MALDI-TOF). Cette analyse est intéressante car elle permet l'étude du mécanisme d'excision. En revanche, elle n'est absolument pas adaptée à une analyse en routine d'une activité enzymatique.

L'excision des bases modifiées peut être aussi suivie par chromatographie gazeuse couplée à la spectrométrie de masse. La démonstration a été réalisée à partir de l'excision de la 5-hydroxy-5,6-dihydrothymine et de la 5,6-dihydrothymine par l'endonucléase III sur de l'ADN irradié par rayonnement y. Cette technique précise présente toutefois plusieurs inconvénients : elle demande un investissement en matériel analytique important, est peu sensible, nécessite beaucoup de matière première et n'est pas adaptée à des analyses de routine.

### Exposé de l'invention.

L'invention a précisément pour objet un procédé de détection et de caractérisation d'activités de protéines impliquées dans la réparation de dommages de l'ADN, plus facile à mettre en oeuvre, adaptable aux différents modes de réparation, utilisable en routine de manière aisée et rapide, en évitant l'emploi de la radioactivité.

Selon l'invention, le procédé de détection et de caractérisation d'une ou plusieurs activité(s) de protéine(s) impliquée(s) dans la réparation de l'ADN, comprend les étapes suivantes:
a) fixer sur un support solide à divers endroits définis plusieurs ADN endommagés constitués par des oligonucléotides de séquence identique présentant des lésions connues différentes et/ou par des oligonucléotides de séquence différente présentant des lésions connues identiques ou différentes,
b) soumettre cet ADN endommagé à l'action d'une composition de réparation contenant ou non au moins une protéine intervenant pour la réparation de cet ADN endommagé, et
c) déterminer l'activité de cette (ces) protéine (s) pour la réparation en mesurant la variation du signal émis par un marqueur qui se fixe sur ou s'élimine du support lors de l'étape b).

Dans ce procédé, on fixe sur un support solide tel qu'une biopuce, l'ADN endommagé dont on connaît au moins une lésion, puis on le soumet à l'action d'une protéine telle qu'une enzyme de réparation, ou d'une composition qui pourrait contenir cette protéine, et on suit la réparation au moyen d'un marqueur qui peut être fixé au départ sur l'ADN endommagé ou introduit dans celui-ci lors du processus de réparation.

Ainsi, le procédé peut servir à caractériser les protéines impliquées dans la réparation de l'ADN. Il peut aussi être utilisé pour mettre en évidence la non-fonctionalité de certaines protéines vis-à-vis de lésions connues, ou encore pour détecter l'absence de protéines de réparation de l'ADN dans des compositions qui normalement devraient en contenir.

L'obtention de ces résultats peut être utile pour des applications en diagnostic. En effet, dans certaines maladies, des gènes de la réparation sont mutés et l'activité enzymatique ou la protéine associée ne sont pas fonctionnelles ou le sont partiellement (xeroderma pigmentosum par exemple).

Dans ce procédé le support solide comprend au moins un site de fixation défini d'un fragment d'ADN endommagé et avantageusement plusieurs sites de fixation permettant l'immobilisation de différents fragments d'ADN endommagés préalablement choisis.

Les protéines dont on veut mesurer l'activité sont les protéines impliquées dans le processus de réparation qui comporte généralement une reconnaissance du dommage, une incision de la chaîne d'ADN, une excision du dommage ou de fragments d'acides nucléiques, une synthèse in situ de l'ADN, et une ligation du brin néoformé.

A titre d'exemple, les protéines impliquées dans ce processus peuvent être choisies parmi:
- les protéines impliquées dans la reconnaissance des lésions telles que XPA, le facteur de transcription TFIIH et les polypeptides qui le composent, XPC, XPF, XPG et leurs protéines associées (famille ERCC, etc)), HSSB, etc (voir Sancar, A. (1995) Annu. Rev. Genetics, 29, pages 69-105, [10]),
- les protéines impliquées dans l'excision des lésions telles que les glycosylases,
- les protéines impliqués dans la resynthèse du (des) nucléotide()s du brin excisé telles que les polymérases, et
- les protéines impliquées dans la ligation de brins néoformés telles que les ligases.

Selon un premier mode de réalisation du procédé de l'invention, le marqueur est présent sur l'ADN endommagé fixé sur le support et il est éliminé par l'action de la protéine dans l'étape b).

Ce mode de réalisation peut être utilisé par exemple pour déterminer l'activité d'enzymes d'incision ou d'excision des lésions d'ADN endommagé. Dans ce cas, le marqueur peut être présent sur une extrémité de l'ADN endommagé. Ainsi, une incision ou une excision provoque l'élimination du fragment d'ADN endommagé portant le marqueur et la perte du signal sur le support à l'endroit où est fixé l'ADN endommagé.

Le premier mode de réalisation peut également être mis en oeuvre avec un marqueur spécifique des lésions de l'ADN endommagé tel qu'un anticorps, qui révèle ces lésions avant l'étape b). Après action de la protéine et réparation des lésions, ce marqueur ne peut se fixer et on peut observer une perte du signal du marqueur représentative de l'activité de la protéine de réparation.

Ainsi, le rapport du signal émis par les anticorps spécifiques avant la réparation de l'ADN sur le signal émis par les anticorps après réaction de la protéine, par exemple réaction enzymatique, est corrélé au taux de réparation de l'ADN. Dans ce dernier cas, la réparation du dommage est évaluée par la disparition d'un signal spécifique du dommage.

Selon un second mode de réalisation du procédé de l'invention, le marqueur est présent dans la composition de réparation et est introduit dans l'ADN fixé sur le support pendant l'étape b).

Dans ce cas, on peut par exemple ajouter dans la composition de réparation, des nucléotides modifiés incorporés par les polymérases et pouvant servir au marquage du brin néoformé. Ces nucléotides modifiés peuvent porter une biotine, un haptène, un composé fluorescent ou toute autre molécule compatible avec le marquage des acides nucléiques. Ces marqueurs introduits lors de l'étape de resynthèse peuvent être ensuite détectés et le signal correspondant corrélé à un taux de réparation.

Les marqueurs utilisables pour déterminer l'activité des protéines impliquées dans la réparation de l'ADN, peuvent être de différents types du moment qu'ils émettent un signal détectable ou puissent être révélés en émettant un signal détectable.

Plusieurs marqueurs ou mode de révélation peuvent être utilisés simultanément ou de façon séquentielle de façon à mettre en évidence des changements d'état intervenus sur les fragments d'ADN endommagés, par les activités protéiques liées à la réparation.

Le marqueur peut être en particulier une molécule d'affinité, un composé fluorescent, un anticorps, un haptène ou une biotine.

De préférence, selon l'invention, on utilise comme marqueur ou révélateur du marqueur, des composés fluorescents à fluorescence directe ou à fluorescence indirecte. De telles molécules peuvent être par exemple l'avidine, révélée par la streptavidine-phycoérithrine, des cryptates d'europium, des composés fluorescents tels que la fluorescéines, la rhodamine, etc...

Les propriétés de transfert d'énergie entre molécules fluorescentes peuvent être également exploitées pour quantifier une activité enzymatique impliquée dans la réparation sur un oligonucléotide substrat. Dans le cas où l'oligonucléotide comportant la ou les bases lésées, porte également une molécule fluorescente autorisant un transfert d'énergie avec une autre molécule fluorescente située soit sur le même oligonucléotide, soit sur un oligonucléotide en contact avec le premier, l'émission d'un signal après excitation prouve la présence de la lésion sur le support. L'incision de l'oligonucléotide comportant la ou les lésions par une enzyme de réparation conduit à l'élimination du fragment incisé et à la perte du signal fluorescent, le transfert d'énergie ne pouvant avoir lieu. Ce changement de signal peut être mesuré et corrélé avec un taux de digestion spécifique.

Selon l'invention, la fixation de l'ADN endommagé sur le support solide peut être effectuée par des procédés classiques.

Ainsi, on peut synthétiser directement sur le support solide un ADN endommagé au moyen de synthétiseurs automatiques permettant par exemple l'incorporation de bases modifiées.

On peut aussi fixer l'ADN endommagé sur le support solide par toute méthode compatible avec le maintien de l'intégrité du fragment d'ADN contenant les dommages, par exemple par hybridation avec un autre fragment d'ADN immobilisé sur le support, par immobilisation par une molécule d'affinité, ou par fixation directe sur la puce par dépôt ou tout autre moyen.

De préférence, on utilise comme support solide une biopuce sur laquelle sont fixés, à des endroits déterminés, des fragments d'ADN comportant des lésions, identiques ou différentes, parfaitement identifiés, ou comportant des lésions multiples non parfaitement identifiées.

Les fragments d'ADN comportant les lésions et immobilisés peuvent être de courts oligonucléotides (15-100 bases de long, de préférence 15 à 50 bases de long) ou des fragments plus longs (100-20 000 bases). Différentes méthodes existent rendant possible l'incorporation d'oligonucléotides comportant des lésions dans de longs fragments d'ADN: Réaction de Polymérisation en Chaîne (PCR), ligation. Les fragments d'ADN immobilisés peuvent être sous forme de simple ou de double brin. La fixation de l'ADN modifié peut se faire directement ou via une molécule intermédiaire (type biotine, antigène, acide nucléique, etc.).

Les longs fragments d'ADN peuvent être également endommagés par tout traitement physique ou chimique induisant des lésions. Par exemple, ils peuvent être irradiés par rayonnement radioactif, solaire ou ultraviolet. Ils peuvent être endommagés par des réactions de photosensibilisation, par des agents chimiques, par des carcinogènes. Dans ce cas, les lésions seront induites statistiquement le long de la chaîne nucléotidique. La qualité, la spécificité et la quantité des lésions induites dépend du choix de l'agent chimique ou physique provoquant les lésions. On peut ainsi cibler la catégorie de lésions que l'on souhaite introduire dans un fragment d'ADN.

Le support peut être par exemple une biopuce comportant des fragments d'ADN, sur laquelle on fixe par hybridation l'ADN endommagé au moyen d'un oligonucléotide comportant une partie complémentaire de l'ADN endommagé et une partie complémentaire de l'un des fragments d'ADN de la biopuce.

L'invention bénéficie entre autre, des avantages de la synthèse chimique d'oligonucléotides et donc du fait que l'on peut choisir exactement les séquences contenant et environnant le dommage.

Le même support peut être fonctionnalisé par des fragments nucléotidiques de séquences identiques ou différentes. Les dommages peuvent être localisés à divers endroits définis ou non des séquences.

La nature des fragments fixés sur le support (taille, nombre de dommages, emplacement des dommages...) est modulable et dépend de la nature des informations recherchées.

Les fragments d'ADN comportant les lésions ou bien les fragments complémentaires des fragments comportant les lésions peuvent également comporter des marqueurs utiles pour la détection des transformations de l'ADN liées à une activité enzymatique de réparation. Ces marqueurs peuvent être des molécules d'affinité, des composés fluorescents, ou tout système de détection spécifique lié à un type de puce spécifique.

Lorsque les fragments d'ADN sont immobilisés sur la puce (puce de référence), une révélation est réalisée permettant de caractériser les états dans lesquels se trouvent les fragments. Les différents plots de la puce sont ainsi caractérisés par les signaux qu'ils émettent. Ils servent de signal de référence.

Ensuite, la puce de référence ou bien une puce comportant des fragments d'ADN endommagés identiques à ceux de la puce de référence, dite puce de test, est incubée en présence de solutions susceptibles de contenir les activités enzymatiques de réparation. Les fragments d'ADN modifiés fixés sur la puce sont susceptibles d'être transformés par les activités enzymatiques présentes.

Une deuxième lecture de la puce de test ou de la puce de référence est réalisée dans des conditions appropriées et après les étapes de révélation nécessaires. Les plots de la puce peuvent ainsi émettre des signaux différents de la première lecture, signifiant qu'une ou plusieurs réactions enzymatiques ont eu lieu sur la puce.

Il est à noter que la mesure de la transformation des ADN endommagés par les enzymes liées aux systèmes de réparation peut être suivie par enregistrement continu des signaux émis par les différents plots de la puce, si les marqueurs utilisés sont compatibles avec un tel système de détection.

Le procédé de l'invention peut être utilisé pour l'étude d'enzymes ou de facteurs protéiques purifiés, ainsi que pour l'étude d'activités présentes dans des extraits cellulaires.

Il peut être utilisé également pour étudier les spécificités de différentes lésions de l'ADN pour certaines enzymes de réparation, en particulier pour les glycosylases. Dans le même domaine, on peut suivre les cinétiques de réparation de ces lésions en fonction de différents facteurs. Ces facteurs peuvent être liés à la séquence nucléotidique (effet des bases environnantes sur la réparation par exemple), à des inhibiteurs ou au contraire à des stimulateurs d'activités réparatrices.

Le suivi de la réparation d'une lésion identifiée est intéressant pour le dosage d'activités enzymatiques impliquées dans les étapes de reconnaissance et d'excision d'une lésion particulière. Par exemple, on sait qu'il existe des glycosylases spécifiques de l'excision d'une lésion particulière. Une déficience d'une enzyme particulière conduit donc à la non réparation d'une lésion particulière.

La miniaturisation du support permet d'améliorer la sensibilité du système en particulier lors de l'utilisation d'extraits cellulaires. L'invention facilite la détection des différentes protéines liées aux systèmes de réparation à partir de cellules en culture ou de biopsies. Par ailleurs, en utilisant une biopuce comportant plusieurs séquences d'ADN endommagé présentant des lésions différentes connues, on peut détecter les capacités de réparation de ces différents dommages pour une quantité limitée d'un même extrait cellulaire. Ceci est très important, notamment dans le cas de diagnostic de certaines pathologies et maladies génétiques.

L'invention présente notamment de nombreux avantages.

En effet, elle permet la miniaturisation des systèmes de dosage d'activités enzymatiques, et plus particulièrement ceux liés à la réparation. Il en résulte un gain de sensibilité. Cette amélioration est très importante puisque les extraits cellulaires sont difficiles à obtenir, en particulier lorsqu'ils proviennent de prélèvements biologiques et non pas de lignées cellulaires.

Elle permet d'obtenir des informations précises sur la fonctionnalité, l'induction et la répression des différentes étapes liées à la réparation des dommages de l'ADN dans des systèmes procaryotes ou eucaryotes.

Elle permet ainsi d'accéder plus facilement à des dosages biologiques d'activités enzymatiques chez l'homme. En particulier, elle permet la détection de déficiences enzymatiques telles qu'on les trouve dans des pathologies faisant intervenir la radiosensibilité ou la photosensibilité comme le Xeroderma pigmentosum, la maladie de Cockaine. Elle permet d'étudier plus facilement les effets du vieillissement sur les activités enzymatiques de réparation.

L'invention permet aussi d'étudier le comportement d'enzymes simultanément envers différentes lésions incorporées dans des fragments nucléotidiques immobilisés sur le même support miniaturisé. Ceci facilite la comparaison des résultats entre eux et les rend plus fiables et reproductibles. Il en résulte également un gain de temps important.

L'invention apporte pour la première fois une approche globale de l'évaluation de la fonctionnalité des systèmes de réparation avec accès à plusieurs lésions et plusieurs mécanismes ou étapes de réparation simultanément.

Une analyse directe des signaux émis par les différents plots de la puce après action des systèmes enzymatiques à étudier, permet de remonter directement aux étapes des différents systèmes de réparation et de conclure sur leur fonctionnement.

L'invention permet le dosage d'une protéine particulière impliquée dans la réparation d'une lésion ciblée, comme par exemple le dosage d'une glycosylase impliquée dans l'excision des bases oxydées. Dans ce cas, la lésion connue pour être le substrat préférentiel de la glycosylase ciblée sera incorporée dans un oligonucléotide de synthèse et fixée sur la puce à un endroit déterminé. On voit donc que l'on peut, en fixant plusieurs oligonucléotides comportant des lésions ciblées et différentes en des endroits différents de la puce, avoir une évaluation de la fonctionnalité des différentes glycosylases impliquées dans le système de REB. Mais, l'invention permet également le dosage de protéines impliquées dans des étapes communes à la réparation de toutes les lésions comme les polymérases et les ligases. Dans ce dernier cas on peut utiliser des fragments d'ADN comportant des lésions moins précisément identifiées.

L'invention se caractérise également par le fait que le choix des différents substrats fixés en des plots différents de la puce permet de cibler le système de réparation auquel on s'intéresse. En effet, la fixation d'oligonucléotides courts (inférieurs à 25 bases) comportant des lésions permet de cibler les protéines du système de REB. La fixation de fragments plus longs (supérieurs à 50 bases) comportant les lésions permet de cibler à la fois les protéines du système REB et REN. La sélection précise des dommages fixés permet également de cibler le systèmes de réparation à étudier.

Ainsi, on peut utiliser le procédé de l'invention pour l'étude de la spécificité et la fonctionnalité des protéines impliquées dans la réparation vis-à-vis de lésions connues d'ADN, pour suivre la cinétique de réparation de lésions de l'ADN par des protéines, pour évaluer l'effet génotoxique de substances ou d'agents physiques inhibant ou stimulant la synthèse des protéines impliquées dans la réparation de l'ADN, ou pour le diagnostic de déficiences des protéines de la réparation liées à des maladies.

D'autres caractéristiques et avantages de l'invention apparaîtront mieux à la lecture des exemples suivants donnés bien entendu à titre illustratif et non limitatif en référence aux dessins annexés.

### Brève description des dessins

La figure 1 représente schématiquement l'état d'un système dans la première étape du procédé de l'invention.

La figure 2 représente schématiquement l'état du système de la figure 1 après l'étape b) du procédé de l'invention.

### Description détaillée des modes de réalisation

### Exemple 1

Dans cet exemple, on utilise le premier mode de réalisation du procédé de l'invention pour déterminer une activité de réparation de type glycosylase sur un oligonucléotide endommagé comportant comme lésion une 8-oxo-7,8 dihydro-2'-désoxyguanosine.

On utilise une biopuce de type MICAM pb4 obtenue comme il est décrit par Livache et al, Biosens. Bioelectron, 13, 1998, page 629 et 634 [8], comportant quatre plots fonctionnalisés par quatre oligonucléotides de synthèse de séquences différentes (H, I, J, K). La séquence H est la suivante :
Séquence H : 5' TTTTT CCA CAC GGT AGG TAT CAG TC

La partie fonctionnalisée de la biopuce est incubée en présence d'un hybride formé de l'oligonucléotide (O) endommagé, comportant une 8-oxo-7,8-dihydro-2'-désoxyguanosine, et d'un oligonucléotide (cOcH) comportant une partie complémentaire de l'oligonucléotide O (cO) et une partie complémentaire de l'oligonucléotide H fixé sur la puce (cH). L'oligonucléotide O comporte une biotine à son extrémité 3'.
O : 5'GAA CTA GTG XAT CCC CCG GGC TGC - Biotine 3'
   (X étant la 8-oxo-7,8 dihydro-2'-désoxyguanosine).
cOcH : 5' GCA GCC CGG GGG ATC CAC TAG TTC GAC TGA CTA CCG TGT GG

L'hybride O-cOcH est obtenu par incubation pendant 60 min à37°C de 10 pmoles de cOcH et 12 pmoles de O dans 200 µl de tampon PBS contenant 0,2 M de NaCl. 20 µl de cette solution sont prélevés et ajoutés sur la partie fonctionnalisée de la puce qui forme une cuvette. Après 1 heure d'incubation en milieu humide à 37°C, la puce est trempée dans un tampon de lavage (PBS/NaCl 0,2M/tween 20 0,05 %).

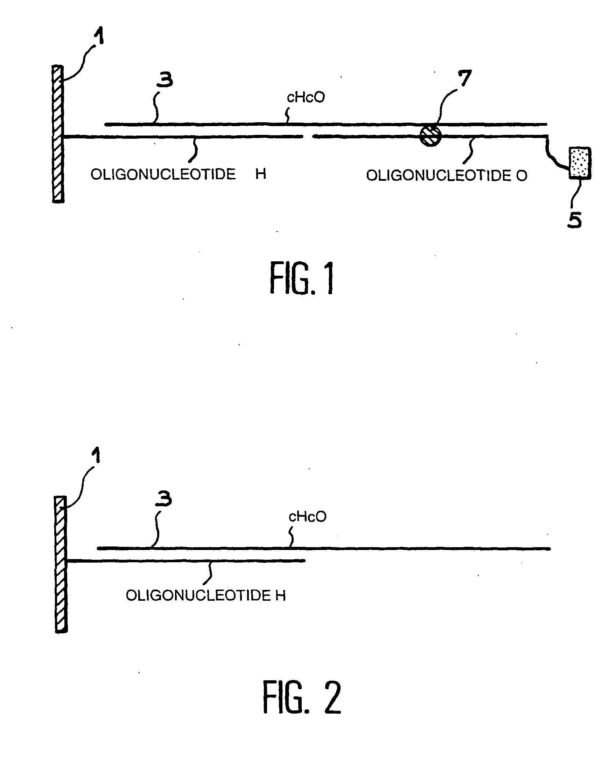

La figure 1 représente le système obtenu par fixation de l'oligonucléotide O sur le plot 1 de la biopuce comportant l'oligonucléotide H. Cet oligonucléotide H est hybridé avec la séquence cH de l'oligonucléotide 3 qui comporte également une séquence hybridée avec l'oligonucléotide endommagé O portant une biotine 5 à son extrémité 3' et une lésion 7.

L'oligonucléotide O comportant la lésion et fixé sur la biopuce par hybridation, est révélé après incubation pendant 10 min à température ambiante avec 20 µl de tampon PBS/NaCl contenant 0,5 % de sérum albumine bovine BSA et 1 µl de streptavidine-phycoérythrine R (0,5 mg/ml ; Jackson ImmunoResearch).

Après lavage dans PBS/NaCl/tween, la biopuce est observée sous microscope à fluorescence et le signal est analysé avec le logiciel Image Pro Plus.

Le signal est intégré en pixels. Les plots fonctionnalisés émettent un léger signal fluorescent même en l'absence de toute hybridation. Les plots non fonctionnalisés sont noirs.

On observe sur le plot fonctionnalisé par l'oligonucléotide H un signal fluorescent intense saturant à 250 pixels. Pour comparaison le plot fonctionnalisé par la séquence I, présente une fluorescence maximale d'environ 110 pixels. Le rapport signal sur bruit (H/I) est de 2,27.

L'hybridation de l'oligonucléotide comportant une lésion de l'ADN est donc spécifique. De plus, on voit qu'il est possible de fixer un fragment d'acide nucléique comportant un dommage donné à un site prédéfini sur une biopuce.

La biopuce est lavée avec NaOH 0,1 N pendant 10 min, puis rincée 10 min avec H₂O, ce qui a pour effet d'éliminer les oligonucléotides O et cOcH de la biopuce. On vérifie au microscope que tout signal a disparu.

La puce est à nouveau hybridée avec l'hybride O-cOcH dans les conditions définies précédemment. Après lavage au PBS/NaCl/tween, la puce est équilibrée 10 min avec un tampon KCl 0,1 M, Tris, puis 20 µl de ce même tampon contenant 0,5 µg de Fapy DNA Glycosylase (S. Boiteux, CEA Fontenay-aux-Roses, France) sont ajoutés. La puce est incubée 30 min à 37°C en atmosphère humide.

Après lavage en PBS/NaCl/tween, on procède comme précédemment à l'étape de révélation avec la streptavidine-phycoérythrine R. Le signal est enregistré après lavage au PBS/tween. La fluorescence intense sur le plot H a disparu. Le rapport signal sur bruit (H/I) est de 1,04.

La figure 2 représente l'état du système après réaction avec l'enzyme de type glycosylase.

Sur cette figure, on voit que l'oligonucléotide 0 a été éliminé de la biopuce. En effet, l'enzyme Fapy DNA glycosylase a coupé l'oligonucléotide O au niveau de la 8-oxo-7,8-dihydro-désoxyguanosine. Les courts fragments d'ADN ainsi engendrés forment des hybrides instables dans les conditions utilisées et sont éliminés de la biopuce. Ceci entraîne la disparition du signal fluorescent dû à la révélation de la biotine.

Cet exemple montre ainsi qu'une activité de réparation de l'ADN est détectable sur un microsupport portant un fragment d'ADN comportant une lésion d'une base de l'ADN.

### Exemple 2

Cet exemple illustre la détection d'une activité de type glycosylase dans un lysat cellulaire en utilisant le premier mode de réalisation du procédé de l'invention.

Dans cet exemple, l'oligonucléotide O utilisé dans l'exemple 1, comportant la 8-oxo-7,8-dihydro-2'-désoxynucléotide, est hybridé comme dans l'exemple 1 sur la biopuce pb4, ce qui correspond au système représenté sur la figure 1.

Un lysat cellulaire total est préparé à partir de cellules Hela en culture.

Les cellules sont trypsinées puis lavées dans un tampon PBS. Le culot contenant environ 15.10⁶ cellules est repris par 1 ml de tampon de lyse (Tris-HCl 10 mM pH 7,5, MgCl₂ 10 mM, KCl 10 mM, EDTA 1 mM, contenant 1 pastille pour 10 ml d'antiprotéases «Complete, Mini» (Boehringer Mannheim) et 5 µl de Phénylméthylsulphonyl Fluoride (PMSF, Sigma, 17,4 mg/ml dans l'isopropanol). Les cellules sont broyées, puis le lysat est centrifugé à 4°C, pendant 50 min à 65 000 tours/min dans une ultracentrifugeuse Beckman. Le surnageant est récupéré puis on rajoute 200 µl de glycérol et 20 µl de dithiotréitol 0,1M. Les lysats sont aliquotés et stockés à -80°C.

La biopuce est ensuite incubée avec 20 µl d'extrait cellulaire pendant 45 min à 37°C. La révélation est effectuée comme dans l'exemple 1.

Le signal du plot H est enregistré. Il est de 180 pixels.

L'expérience est renouvelée avec du lysat dénaturé (chauffage à 100°C pendant 10 min). Le signal du plot H est saturant à 250 pixels. On a donc une perte de signal suite à l'incubation de la biopuce fonctionnalisée par le duplex contenant le dommage avec du lysat cellulaire brut. Cette perte de signal est d'environ 30 %, ce qui correspond à la coupure partielle de l'oligonucléotide modifié O par les enzymes contenues dans le lysat.

Ces expériences sont confirmées par analyse sur gel de polyacrylamide après marquage radioactif de l'oligonucléotide O.

### Exemple 3

Cet exemple illustre la détection de l'activité de réparation d'excision/resynthèse d'un lysat cellulaire total, en utilisant un fragment d'ADN modifié fixé sur un microsupport.

Dans ce cas, on utilise le second mode de réalisation du procédé de l'invention.

Un fragment d'ADN de 5000 paires de bases est préparé par amplification PCR à partir du phage lambda en utilisant la trousse «Expand^{™} Long template PCR system» de Roche. Une des amorces d'amplification comporte une séquence de 15 bases (J₁₅) à son extrémité 5' séparée par un synthon amine de la séquence s'hybridant sur le phage. Cette séquence reste simple brin après l'amplification PCR. Ce fragment PCR est purifié par colonne micro-spin S300 (Amersham-Pharmacia). Le brin d'ADN est irradié 3 minutes par des rayons ultra-violets de type C (λₘₐₓ 254 nm, 0,8 J/cm²).

L'ADN irradié est ensuite hybridé par l'intermédiaire d'un oligonucléotide de 30 bases de long cI₁₅cJ₁₅ complémentaire pour une part de la séquence I₁₅ et pour une autre part de la séquence J₁₅, sur une biopuce pb2 (MICAM) comportant quatre séquences oligonucléotidiques différentes sur quatre plots différents (S1, S2, S3, I₁₅), dont la séquence appelée I₁₅.
I₁₅: 5' TTTTT ATC CGT TCT ACA GCC

L'hybridation se déroule 1 heure à 37°C dans 20 µl de tampon PBS/NaCl contenant environ 0,1 pmole du produit d'amplification PCR.

La puce est ensuite incubée en présence d'extraits cellulaires totaux dans un tampon adapté comme décrit ci-dessous.

### Expérience d'excision-resynthèse sur la puce fonctionnalisée:

Le protocole est adapté de Robins et al, The EMBO Journal, vol. 10, n°12, pages 3913-3921, 1991 [10]. Une solution est préparée contenant 25 µl de lysat cellulaire de cellules Hela, 10 µl de tampon réactionnel 5X (Hepes KOH 225 mM pH 7,8, KC1, 350 mM, MgCl₂ 37,5 mM, DTT 4,5 mM, EDTA 2 mM, BSA 0,09 mg/ml, glycérol 17 %), ATP 2 mM, dGTP 5 µm, dATP 5 µM, dCTP 5 µM, dTTP 1 µM, Biotine-16-2'-désoxyuridine-5'-triphosphate 4 µM, phosphocréatine 200 mM, créatine phosphokinase type I 12,5 µg dans un volume total de 50 µl. 25 µl de cette solution sont déposés sur la biopuce qui est incubée 2h30 à 30°C en milieu humide.

Après rinçage au PBS/NaCl/tween, on effectue la révélation avec la streptavidine-phycoérythrine.

La puce est ensuite analysée au microscope.

On observe une forte fluorescence (signal saturant) au niveau du plot fonctionnalisé par l'oligonucléotide I₁₅ Le rapport signal sur bruit (I₁₅/S3) est de 2,2.

Une révélation streptavidine-phycoérythine effectuée avant réparation, par les enzymes contenues dans le lysat, du fragment d'ADN modifié donne un rapport signal sur bruit (I₁₅/S3) de 1.

L'expérience est renouvelée avec le fragment non modifié obtenu par réaction PCR. Aucun signal n'est visible sur la puce après réaction d'excision/resynthèse effectuée dans les conditions précédentes (signal/bruit 1).

Cet exemple montre que les activités enzymatiques impliquées dans la réparation des bases modifiées de l'ADN sont détectables sur une puce fonctionnalisée par un fragment d'ADN comportant des bases modifiées.

### Exemple 4

Cet exemple illustre l'utilisation d'anticorps spécifiques pour la mise en évidence de lésions constituées par des dimères de pyrimidine de type cyclobutane

On utilise la même biopuce que dans l'exemple 3, sur laquelle est fixée l'ADN irradié dans les mêmes conditions que celles de l'exemple 3, au moyen du même oligonucléotide cI₁₅cJ₁₅.

Après lavage en PBS/NaCl/tween, on procède à la révélation des dimères de pyrimidine de type cyclobutane formés par l'irradiation UVB.

La puce est incubée avec 20 µl de tampon PBS/NaCl contenant 1 µl d'anticorps anti-dimère (500 µg/ml; Kamiya Biomedicam Company, Seattle, USA), pendant 1 heure à 37°C.

Après lavage au PBS/NaCl/tween la puce est incubée en présence de 20 µl de PBS/NaCl contenant 1 µl d'anticorps de chèvre anti-souris couplé au marqueur Cy^{™}3 (1,4 mg/ml; Jackson ImmunoResearch Laboratories, Inc.) pendant 1 h à 37°C.

Après lavage au PBS/NaCl/tween, le signal est enregistré sur la puce. Le rapport signal sur bruit (I₁₅/S3) est de 1,33.

Ainsi, l'utilisation d'anticorps anti-dommages de l'ADN permet de détecter spécifiquement des dommages et donc de suivre leur élimination par des enzymes de réparation.

### Références citées.

[1]: Wood et al, Cell, 53, 1988, pages 97-106.
[2]: Sallès et al, dans Biochimie, 77, 1995, pages 796-802.
[3] : FR-A-2 731 711.
[4]: Page et al, Biochemistry, 29, 1990, pages 1016-1024.
[5] : Huang et al, Proc. Natl. Acad. Sci., 91, 1994, pages 12213-12217.
[6] : Romieu et al, J. Org. Chem., 63, 1998, pages 5245-5249.
[7] : D'Ham et al, Biochemistry, 38, 1999, pages 3335-3344.
[8]: Livache et al, Biosens. Bioelectron, 13, 1998, pages 629-634.
[9] : Wilson III, D.M. and Thompson, L.H., 1997, Proc. Natl. Acad. Sci., 94, pages 12754-12757.
[10] : Sancar, A. (1995) Annu. Rev. Genetics, 29, pages 69-105.

## Revendications

1. Procédé de détection et de caractérisation d'une ou plusieurs activité(s) de protéine(s) impliquée (s) dans la réparation de l'ADN, comprenant les étapes suivantes :
a) fixer sur un support solide à divers endroits définis plusieurs ADN endommagés constitués par des oligonucléotides de séquence identique présentant des lésions connues différentes et/ou par des oligonucléotides de séquence différente présentant des lésions connues identiques ou différentes,
b) soumettre cet ADN endommagé à l'action d'une composition de réparation contenant ou non au moins une protéine intervenant pour .la réparation de cet ADN endommagé, et
c) déterminer l'activité de cette (ces) protéine(s) pour la réparation en mesurant la variation du signal émis par un marqueur qui se fixe sur ou s'élimine du support lors de l'étape b).

2. Procédé selon la revendication 1, dans lequel la protéine est choisie parmi les protéines impliquées dans la reconnaissance des lésions de l'ADN, les protéines impliquées dans l'excision des lésions de l'ADN, les protéines impliquées dans la resynthèse du (des) nucléotides du brin excisé et les protéines impliquées dans la ligation de brins néoformés.

3. Procédé selon la revendication 1, dans lequel le marqueur est présent sur l'ADN endommagé fixé sur le support et est éliminé par l'action de la protéine dans l'étape b).

4. Procédé selon la revendication 3, dans lequel le marqueur est fixé à une extrémité de l'ADN endommagé, la protéine est une enzyme d'incision ou d'excision des lésions de l'ADN endommagé, et l'incision ou l'excision provoque l'élimination d'un fragment de l'ADN endommagé portant le marqueur.

5. Procédé selon la revendication 3, dans lequel le marqueur est un marqueur spécifique des lésions de l'ADN endommagé susceptible de révéler ces lésions avant l'étape b) et la lésion est éliminée lors de la réparation de sorte que le marqueur ne peut plus révéler la lésion.

6. Procédé selon la revendication 1, dans lequel le marqueur est présent dans la composition de réparation et est introduit dans l'ADN fixé sur le support pendant l'étape b).

7. Procédé selon la revendication 6, dans lequel la composition de réparation comprend un nucléotide modifié par le marqueur.

8. Procédé selon la revendication 1, dans lequel la composition de réparation est un lysat cellulaire ou une enzyme de réparation purifiée.

9. Procédé selon l'une quelconque des revendications 1 à 8, dans lequel le marqueur est une molécule d'affinité, un composé fluorescent, un anticorps, un haptène ou une biotine.

10. Procédé selon l'une quelconque des revendications 1 à 9, dans lequel le support est une biopuce comportant des fragments d'ADN, sur laquelle on fixe par hybridation l'ADN endommagé au moyen d'un oligonucléotide comportant une partie complémentaire de l'ADN endommagé et une partie complémentaire de l'un des fragments d'ADN de la biopuce.

11. Procédé selon l'une quelconque des revendications 1 à 10, dans lequel l'ADN endommagé est un oligonnucléotide court de 15 à 100 bases de long, de préférence de 15 à 50 bases de long, comportant des lésions incorporées dans l' oligonucléotide lors de sa synthèse chimique.

12. Procédé selon l'une quelconque des revendications 1 à 10, dans lequel l'ADN endommagé est un polynucléotide dé 100 à 20 000 bases de long.

13. Procédé selon l'une quelconque des revendications 1 à 12, dans lequel l'ADN endommagé.est sous forme de simple ou double brin.

14. Biopuce sur laquelle sont fixés à divers endroits définis plusieurs ADN endommagés constitués par des oligonucléotides de séquence identique, présentant des lésions connues différentes et/ou par des oligonucléotides de séquence différente présentant des lésions connues identiques ou différentes.

15. Utilisation du procédé selon l'une quelconque des revendications 1 à 13, pour l'étude de la spécificité et la fonctionnalité des protéines impliquées dans la réparation vis-à-vis de lésions connues d'ADN.

16. Utilisation du procédé selon l'une quelconque des revendications 1 à 13, pour suivre la cinétique de réparation de lésions de l'ADN par des protéines.

17. Utilisation du procédé selon l'une quelconque des revendications 1 à 13, pour évaluer l'effet génotoxique de substances ou d'agents physiques inhibant ou stimulant la synthèse des protéines impliquées dans la réparation de l'ADN.

18. Utilisation du procédé selon l'une quelconque des revendications 1 à 13, pour le diagnostic de déficiences des protéines de la réparation liées à des maladies.

## Claims

1. Method for the detection and characterisation of one or more activity(ies) of protein(s) involved in the repair of DNA, comprising the following stages:
a) Fixing on a solid support, at various defined points, a plurality of damaged DNA samples consisting of oligonucleotides of identical sequence presenting different known injuries and/or of oligonucleotides of different sequence presenting known identical or different injuries,
b) Submitting this damaged DNA to the action of a repair composition, containing or not at least one intervention protein for the repair of this damaged DNA, and
c) Determining the activity of this/these protein(s) for the repair by measuring the variation in the signal emitted by a marker which attaches itself on or is eliminated from the support during stage b).

2. Method according to Claim 1, in which the protein is chosen from among the proteins involved in the recognition of the injuries to the DNA, the proteins involved in the excision of the injuries to the DNA, the proteins involved in the resynthesis of the nucleotide(s) of the excised strand and the proteins involved in the linking of the newly-formed strands.

3. Method according to Claim 1, in which the marker is present on the damaged DNA fixed on the support and is eliminated by the action of the protein in stage b).

4. Method according to Claim 3, in which the marker is fixed at one end of the damaged DNA, the protein is an enzyme for the incision or excision of the injuries to the damaged DNA, and the incision or excision causes the elimination of a fragment of the damaged DNA carrying the marker.

5. Method according to Claim 3, in which the marker is a specific marker for the injuries to the damaged DNA, capable of revealing these injuries before stage b) and the injury is eliminated during the repair in such a way that the marker can no longer reveal the injury.

6. Method according to Claim 1, in which the marker is present in the composition of the repair and is introduced into the DNA fixed on the support during stage b).

7. Method according to Claim 6, in which the composition of the repair includes a nucleotide modified by the marker.

8. Method according to Claim 1, in which the composition of the repair is a cellular lysate or a purified repair enzyme.

9. Method according to any one of Claims 1 to 8, in which the marker is an affinity molecule, a fluorescent compound, an antibody, a haptene, or a biotine.

10. Method according to any one of Claims 1 to 9, in which the support is a biochip comprising fragments of DNA on which the damaged DNA is fixed by hybridation, by means of an oligonucleotide comprising a complementary part of the damaged DNA and a complementary part of one of the DNA fragments from the biochip.

11. Method according to any one of Claims 1 to 10, in which the damaged DNA is a short oligonucleotide of 15 to 100 bases in length, preferably 15 to 50 bases in length, comprising injuries incorporated in the oligonucleotide during its chemical synthesis.

12. Method according to any one of Claims 1 to 10, in which the damaged DNA is a polynucleotide from 100 to 20,000 bases in length.

13. Method according to any one of Claims 1 to 12, in which the damaged DNA is in the form of a single or double strand.

14. Biochip on which are fixed at various defined points a plurality of damaged DNA fragments consisting of oligonucleotides of identical sequence, presenting different known injuries and/or of oligonucleotides of different sequence, presenting known identical or different injuries.

15. Use of the method according to any one of Claims 1 to 13 for the study of the specificity and functionality of the proteins involved in the repair in respect of the known DNA injuries.

16. Use of the method according to any one of Claims 1 to 13 to trace the kinetics of repair of the DNA injuries by proteins.

17. Use of the method according to any one of Claims 1 to 13 to evaluate the genotoxic effect of substances or physical agents which inhibit or stimulate the synthesis of the proteins involved in the repair of the DNA.

18. Use of the method according to any one of Claims 1 to 13 to diagnose the deficiencies of the proteins for the repair associated with illnesses.

## Patentansprüche

1. Verfahren zur Detektion und Charakterisierung einer oder mehrerer Aktivität(en) eines Proteins/von Proteinen, das/die an der Reparatur von DNS beteiligt ist/sind, umfassend die folgenden Schritte:
a) Fixieren mehrerer beschädigter DNS, die aus Oligonucleotiden mit identischer Sequenz, die bekannte, verschiedene Läsionen aufweisen, und/oder aus Oligonucleotiden mit unterschiedlicher Sequenz, die bekannte, identische oder verschiedene Läsionen aufweisen, bestehen, auf einem festen Träger an verschiedenen definierten Stellen;
b) Unterwerfen dieser beschädigten DNS der Einwirkung einer Reparatur-Zusammensetzung, die wenigstens ein Protein enthält oder nicht enthält, das in die Reparatur dieser beschädigten DNS eingreift; und
c) Bestimmen der Aktivität dieses/dieser Protein(e) für die Reparatur unter Messen der Veränderung des Signals, das durch einen Marker emittiert wird, der während des Schritts (b) auf dem Träger fixiert oder von diesem eliminiert wird.

2. Verfahren nach Anspruch 1, in dem das Protein gewählt ist unter den Proteinen, die beteiligt sind an der Erkennung der Läsionen der DNS, den Proteinen, die beteiligt sind an der Exzision der Läsionen der DNS, den Proteinen, die an der Resynthese des/der Nucleotid(e) des herausgeschnittenen Strangs beteiligt sind, und den Proteinen, die an der Ligation der neugebildeten Stränge beteiligt sind.

3. Verfahren nach Anspruch 1, in dem der Marker auf der beschädigten DNS zugegen ist, die auf dem Träger fixiert ist und durch Einwirkung des Proteins von Schritt (b) eliminiert wird.

4. Verfahren nach Anspruch 3, in dem der Marker an einem Ende der beschädigten DNS fixiert ist, das Protein ein Enzym zum Einschneiden oder zum Herausschneiden von Läsionen der beschädigten DNS ist und das Einschneiden oder Herausschneiden die Eliminierung eines Fragments der beschädigten DNS hervorruft, das den Marker trägt.

5. Verfahren nach Anspruch 3, in dem der Marker ein für Läsionen der beschädigten DNS spezifischer Marker ist, der in der Lage ist, diese Läsionen vor dem Schritt (b) aufzudecken, und die Läsion während der Reparatur in der Weise eliminiert wird, dass der Marker die Läsion nicht mehr aufdecken kann.

6. Verfahren nach Anspruch 1, in dem der Marker in der Reparatur-Zusammensetzung zugegen ist und in die auf dem Träger fixierte DNS während des Schritts (b) eingeführt wird.

7. Verfahren nach Anspruch 6, in dem die Reparatur-Zusammensetzung ein Nucleotid umfasst, das durch den Marker modifiziert ist.

8. Verfahren nach Anspruch 1, in dem die Reparatur-Zusammensetzung ein Zell-Lysat oder ein gereinigtes Reparatur-Enzym ist.

9. Verfahren nach einem der Ansprüche 1 bis 8, in dem der Marker ein Affinitäts-Molekül, eine Fluoressenz-Verbindung, ein Antikörper, ein Hapten oder ein Biotin ist.

10. Verfahren nach einem der Ansprüche 1 bis 9, in dem der Träger ein Biochip ist, der DNS-Fragmente umfasst, auf dem man durch Hybridisierung die beschädigte DNS mittels eines Oligonucleotids fixiert, das einen komplementären Abschnitt der beschädigten DNS und einen komplementären Abschnitt eines der Fragmente der DNS des Biochips umfasst.

11. Verfahren nach einem der Ansprüche 1 bis 10, in dem die beschädigte DNS ein kurzes Oligonucleotid mit einer Länge von 15 bis 100 Basen ist, vorzugsweise einer Länge von 15 bis 50 Basen, das die Läsionen in dem Oligonucleotid während seiner chemischen Synthese inkorporiert umfasst.

12. Verfahren nach einem der Ansprüche 1 bis 10, in dem die beschädigte DNS ein Polynucleotid mit einer Länge von 100 bis 20.000 Basen ist.

13. Verfahren nach einem der Ansprüche 1 bis 12, in dem die beschädigte DNS in einzelsträngiger oder doppelsträngiger Form vorliegt.

14. Biochip, auf dem an verschiedenen definierten Orten mehrere beschädigte DNS fixiert sind, die aus Oligonucleotiden mit identischer Sequenz, die bekannte, verschiedene Läsionen aufweisen, und oder aus Oligonucleotiden mit unterschiedlicher Sequenz, die bekannte, identische oder unterschiedliche Läsionen aufweisen, bestehen.

15. Verwendung des Verfahrens nach einem der Ansprüche 1 bis 13 zur Untersuchung der Spezifität und der Funktionalität der Proteine, die an der Reparatur von bekannten Läsionen einer DNS beteiligt sind.

16. Verwendung des Verfahrens nach einem der Ansprüche 1 bis 13 zum Verfolgen der Kinetik der Reparatur von Läsionen der DNS durch die Proteine.

17. Verwendung des Verfahrens nach einem der Ansprüche 1 bis 13 zum Bewerten des genotoxischen Effekts von Substanzen oder physischen Mitteln, die die Synthese der Proteine inhibieren oder stimulieren, die an der Reparatur der DNS beteiligt sind.

18. Verwendung des Verfahrens nach einem der Ansprüche 1 bis 13 zum Diagnostizieren von Mängeln von Reparaturproteinen, die mit Krankheiten verbunden sind.


### LISTE DE SEQUENCES

<110> COMMISSARIAT A L'ENERGIE ATOMIQUE SAUVAIGO, Sylvie
<120> DETECTION ET CARACTERISATION DE L'ACTIVITE DE PROTEINES IMPLIQUEES DANS LA REPARATION DE LESIONS DE L'ADN
<130> B13548MDT
<140> PCT/FR01/01605
   <141> 2001-05-23
<150> FR00/06624
   <151> 2000-05-24
<160> 4
<170> PatentIn Ver. 2.1
<210> 1
   <211> 24
   <212> ADN
   <213> Séquence artificielle
<220>
   <223> Description de la séquence artificielle: oligonucléotide de synthèse
<400> 1
   tttttccaca cggtaggtat cagtc 25
<210> 2
   <211> 24
   <212> ADN
   <213> Séquence artificielle
<220>
   <223> Description de la séquence artificielle: oligonucléotide de synthèse
<220>
   <221> modified_base
   <222> (10)
   <223> 8-oxo-7,8 dihydro-2'-désoxyguanosine
<220>
   <223> -biotine 3'
<400> 2
   gaactagtgn atcccccggg ctgc 24
<210> 3
   <211> 41
   <212> ADN
   <213> Séquence artificielle
<220>
   <223> Description de la séquence artificielle: oligonucléotide de synthèse
<400> 3
   gcagcccggg ggatccacta gttcgactga ctaccgtgtg g 41
<210> 4
   <211> 20
   <212> ADN
   <213> Séquence artificielle
<220>
   <223> Description de la séquence artificielle: oligonucléotide de synthèse
<400> 4
   tttttatccg ttctacagcc 20